# EUROPEAN PATENT SPECIFICATION

(11) **EP 0 353 316 B1**
(45) Date of publication and mention of the grant of the patent: **13.10.1993**
(21) Application number: 88112449.9
(22) Date of filing: 01.08.1988
(51) Int. Cl.: A61B 5/02

(54) **Method for measuring blood pressure and apparatus for automated blood pressure measuring**
Verfahren und Vorrichtung zur automatischen Blutdruckmessung
Procédé et appareil pour la mesure automatique de la pression sanguine

(43) Date of publication of application: 07.02.1990
(73) Proprietor: Hewlett-Packard GmbH, D-71004 Böblingen (DE)
(72) Inventor: Frankenreiter, Michael, D-7032 Sindelfingen (DE)
(74) Representative: Kurz, Peter

(56) References cited:
- US-A- 4 360 029

## Description

The present invention relates to a method for measuring blood pressure, comprising the steps of applying a blood pressure cuff about a subject's limb containing an artery; inflating said cuff to a pressure pₘₐₓ above the systolic pressure p_{sys}, thereby occluding said artery, reducing cuff pressure from pₘₐₓ to at least a pressure pₘᵢₙ below the diastolic pressure p_{dia}, thereby permitting an increasing flow through the progressively less occluded artery, detecting of effects at the cuff caused by said increasing flow through that progressively less occluded artery, processing said detected effects in processing means and displaying said processed effects as subject's actual blood pressure values, and conducting a next subsequent blood pressure measuring cycle after the end of a death-time interval Δt_{dea}, said death time interval starting at a time t_{dz} after cuff pressure reaching pₘᵢₙ.

The invention further relates to an apparatus for automated blood pressure measuring, in particular for cyclic measuring, comprising an inflatable and deflatable pressure cuff, said cuff being applicable about a subject's limb containing an artery, means for inflating said cuff to a pressure pₘₐₓ above the systolic pressure p_{sys}, thereby occluding said artery, means for reducing cuff pressure from pₘₐₓ to at least a pressure pₘᵢₙ below the diastolic pressure p_{dia}, thereby permitting an increasing flow through the progressively less occluded artery, means for detecting of effects at the cuff caused by said increasing flow through the progressively less occluded artery, processing means for processing said detected effects and for displaying said processed effects as subject's actual blood pressure values, said processing means further comprising means for retarding the start of a next subsequent measuring cycle until a death-time interval Δt_{dea} has ended.

Such so-called "non-invasive" blood pressure measuring methods and apparatus are well-known in the art and known as sphygmomanometers. A similar method and a similar apparatus is known from EP-A2-0 207 807.

During such non-invasive sphygmanometric measurements, an inflatable cuff is suitably located around a limb of a subject, for example a human, and is inflated up to a predetermined pressure pₘₐₓ above the systolic pressure p_{sys}, thereby occluding an artery. The limb may be an arm, in particular the upper arm, a foot, or a finger of subject. Thereupon, cuff pressure is reduced, thereby permitting an increasing flow through the progressively less occluded artery. The effects at the cuff may be detected by the auscultative method, thereby detecting the so-called "Korotkoff noise" caused by the artery during deflating of cuff.

A further well-known method is the so-called "oscillometric method", for example as described within EP-A2-0 208 520.

The oscillometric method of measuring blood pressure is one of the most popular methods in commercially available systems. This method relies on measuring changes in arterial counterpressure, such as imposed by the inflatable cuff which is controllably relaxed or inflated. The cuff pressure is reduced in predetermined increments, and at each pressure level fluctuations are monitored. The resulting signals typically consist of the DC voltage with small superimposed variational component caused by arterial blood pressure pulsations. After suitable filtering to reject the DC component and to provide amplification, peak pulse amplitudes above a given base line are measured and stored. As deflating of cuff continues, the peak amplitudes will normally increase from a lower level to a relative maximum, and thereafter will decrease. The lowest cuff pressure at which the oscillation has a maximum value is representative of mean arterial pressure. Systolic and diastolic pressures can be derived as a predetermined fraction of mean arterial pressure, or by more sophisticated methods of direct processing of the oscillatory complexes.

In some applications, several subsequent measurement cycles are necessary and become essential aspect of human and veterinary treatment. Such subsequent measurements are preferably conducted in emergency rooms, intensive and critical care units, and in the operating theatre.

One problem on conducting subsequent measurement cycles is the dimension of the pause or death-time between two subsequent cycles. Death-time is understood as an interval starting from a time at which the deflated cuff has reached a minimum pressure, for example 666 Pa (5 mmHg), at which minimum pressure the cuff is deflated approximately totally, and ending at the start time tₙₑₓₜ of the next subsequent cycle.

The known methods and the known apparatus for conducting said measurements in a cyclic manner, so-called "stat-mode", use a constant predetermined death-time interval.

If the death-time interval is chosen long, this may be agreeable to the patient. Long death-time intervals, however, do not allow a lot of measurement cycles within a predetermined time interval. For example, the effects of a drug, applicated to the patient during a surgical operation need to be supervised by blood measurement cycles with 20 or 30 seconds.

If using a very short death-time interval, it may be very disagreeable for the patient. Furthermore, if a overall time of a measurement cycle is very long, for example based on artifacts during measurement caused by movements of the patient, a short death-time interval is not convenient because of possible blood backwash reflecting to the heart of the patient. Time-consuming measurement cycles are necessary, if patient's systole and diastole are far apart.

Nevertheless, all known methods and apparatus for automated non-invasive blood pressure measurements use constantly preselected death-time intervals between two subsequent measurement cycles.

It is, therefore, object of the present invention to provide a method and an apparatus for measuring blood pressure as initially indicated using death-time intervals being convenient to the individual subject to be measured.

This object is achieved by a method, comprising the step of starting said next subsequent blood pressure measuring cycle at a time tₙₑₓₜ next being a function of a cycle specific time interval Δtₚᵣₑ of the preceding measuring cycle. This object is further achieved by an apparatus with means for retarding start of next cycle, said means comprising time-incrementing means for storing a cycle specific time interval Δtₚᵣₑ of a preceding measuring cycle, said retarding means allow a start of a next subsequent cycle as a function of said time interval Δtₚᵣₑ.

According to the invention, each death-time interval is adapted to the individual subject to be measured and is adapted to the preceding measuring cycle during a cyclic measurement, in particular in stat-mode measurements.

The cycle specific time interval Δtₚᵣₑ may be the overall time for conducting said preceding measuring cycle. In particular, the death-time interval may be a fraction of the overall time interval. As a result, a short time interval Δtₚᵣₑ leads to a short death-time interval, and on the contrary, a long time interval Δtₚᵣₑ leads to a long death-time interval. Further, if the cycle specific time interval Δtₚᵣₑ varies during several measurement cycles, for example in response to drugs applied to patient, the death-time interval is automatically adapted cycle by cycle to the varying preceding measuring cycle. The apparatus according to the invention automatically stores time increments of a preceding cycle and allows a start of a next subsequent cycle after a death-time interval being a function of said time increments.

So, according to the invention, both a death-time interval adaption to the individual subject to me measured and an automatic adaption to varying measurement cycle intervals is achieved. As a result, blood pressure measuring is more agreeable to subject.

According to another aspect of the invention, the time interval tₚᵣₑ starts at a time t₁ after reaching a threshold cuff pressure pₜₕᵣ during inflating said cuff, and ends at a time t₂ after reaching the said threshold pressure pₜₕᵣ again during the cuff pressure reducing step of said cycle.

This has the advantage that the time interval Δtₚᵣₑ depends on the varying cuff pressures during measuring cycle, said varying cuff pressures are essential factors to which the measuring cycle time depends. The time interval tₚᵣₑ corresponds to this time the cuff pressure being above a threshold cuff pressure. The death-time interval may be, for example, elected as identical with the time interval Δtₚᵣₑ.

According to another aspect of the invention, the said threshold pressure Δtₚᵣₑ corresponds to the mean blood pressure pₘₑₐ of the subject to be measured.

This has the advantage that the threshold pressure is adapted to a particular blood pressure value being representative to the actual condition of the subject to be measured. As a result, a subsequent next cycle depends on both an absolute time interval of the preceding cycle and to the blood pressure measured during said preceding cycle.

According to another aspect of the invention, the threshold cuff pressure pₜₕᵣ varies within

${\text{p}}_{\text{thrmin}} {\text{≦ p}}_{\text{thr}} {\text{≦ p}}_{\text{thrmax}} \text{.}$

This has the advantage, that, in particular if the threshold cuff pressure pₜₕᵣ corresponds to the mean pressure pₘₑₐ of the subject to be measured, pₜₕᵣ cannot exceed minimum and maximum limits. This assures that if an extremely shift of the mean blood pressure pₘₑₐ occurs, the corresponding death-time interval neither becomes too short nor unnecessary long.

According to another aspect of the invention, for measuring the blood pressure of a human, the threshold cuff pressure pₜₕᵣ is limited to

${\text{p}}_{\text{thrmin}} {\text{= 6,6 kPa(50 mmHg) ≦ p}}_{\text{thr}} {\text{≦ p}}_{\text{thrmax}} \text{= 13,33 kPa(100 mm Hg).}$

This has the advantage that the threshold pressure varies within an area being typical for human mean blood pressure values. For the first measuring cycle, a preselected threshold cuff pressure is used, since within said first cycle the mean blood pressure value is not yet known.

According to another aspect of the invention, a further cycle is started if a second time interval ${\text{Δt}}_{\text{preII}} {\text{= Δt}}_{\text{pre}}$ following to first time interval Δtₚᵣₑ has ended.

This has the advantage that a very simple method is achieved, and a simple equipment of an apparatus according to the invention is possible. A first counter is provided, said first counter starting incrementing after reaching at a time t₁ the threshold cuff pressure pₜₕᵣ during inflating cuff. Said first counter starts decrementing after reaching at a time t₂ the said threshold pressure pₜₕᵣ again during the cuff pressure reducing step of the said cycle. So, a subsequent next measuring cycle is started if first counter is zero.

According to another aspect of the invention, said second cycle is started if both said second time interval Δt_{preII} and a minimum death-time interval Δtₘᵢₙ have ended.

In some cases, if tₚᵣₑ is a very short interval, it may occur that after end of the second time interval Δt_{preII} the cuff pressure has not yet reached pₘᵢₙ. A deflation of cuff pressure down to pₘᵢₙ, however, is wanted in view of convenience for subject to be measured. For example, it is more agreeable to patient if the cuff is deflated nearly totally or at least to pₘᵢₙ before inflating the cuff again. The minimum death-time interval Δtₘᵢₙ may be very short and possibly zero, in particular if the second time interval Δt_{preII} has end several seconds before start point time t_{dz} of the death-time interval Δtₘᵢₙ.

According to another aspect of the invention, said second cycle is started if a maximum death-time interval Δtₘₐₓ has ended, but said second time interval Δt_{preII} has not yet ended.

This has the advantage that in cases of very large second time intervals Δt_{preII}, and wherein the start time t_{dz} of the death-time interval is in a earlier phase of the said second time interval Δt_{preII}, the death-time interval is not unnecessary long. If waiting in these cases, until a second time interval Δt_{preII} has gone, a death-time interval of 10 or 15 seconds may occur. This time is unnecessary long and results in a reduced number of measuring cycle units per time unit. For example, a maximum death-time interval of 6 seconds is sufficient for human blood pressure measurements. Within an apparatus according to the invention, this feature is achieved by the fact that the said first counter is connected to a second counter incrementing a preselected maximum death-time interval, said second counter starts incrementing after cuff pressure has reached pₘᵢₙ. So, a start of the next subsequent cycle can occur if first counter has reached zero and meanwhile second counter has incremented to a minimum death-time interval, but the latest if the second counter has incremented to said maximum death-time interval.

Some embodiments of the present invention will not be described with reference to the accompanying drawings in which
- Figure 1: shows a graph having plotted a cuff pressure via time, demonstrating a first embodiment according to the invention,
- Figure 2: shows a similar graph as Figure 1, demonstrating a second embodiment according to the invention,
- Figure 3: shows a graph similar to that of Figures 1 and 2 demonstrating a third embodiment according to the invention, and
- Figure 4: is a flow chart representing the operation of an apparatus according to one embodiment of the invention.

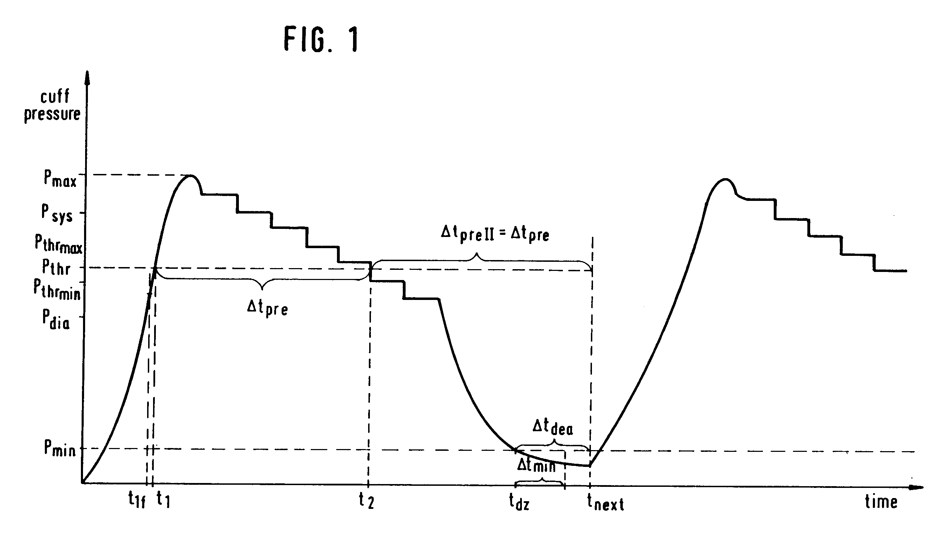

Referring now to Figure 1, the graph shown is representative to a cuff pressure/time graph of an non-invasive blood pressure measurement. A blood pressure cuff is applied about a subject's artery and inflated above the systolic level p_{sys} up to pₘₐₓ, thus fully occluding the artery for a full heart cycle. The cuff pressure is thereafter reduced step by step to permit an increasing flow through the progressively less occluded artery. For example, in accordance with conventional oscillometric techniques, pressure oscillations in the artery are sensed by changes in the counterpressure of the cuff, and in turn by a transducer. A measure of the peak amplitudes of the successively encountered oscillatory complexes (not shown in this graph) are stored in a memory. Also retained is the cuff pressure obtained for each complex peak. As the measurement cycle progresses, the peak amplitude of the blood pressure complexes generally become monotonically larger to a maximum and then become monotonically smaller as the cuff pressure continues toward deflation. The peak amplitude of the cuff pressure oscillation complexes and the corresponding occluding cuff pressure values are retained in a computer memory of a processing means.

The inflating of cuff up to pₘₐₓ occurs within 2 to 4 seconds, depending on cuff size and arrangement of cuff, e.g. about subject's upper arm or finger.

If cuff pressure reaches a threshold cuff pressure value pₜₕᵣ at time t₁, a counter or timer is started, incrementing time up to time t₂. The time t₂ corresponds to the point, the cuff pressure reaches again the threshold value pₜₕᵣ during the stepwise deflation of cuff.

At time t₂, a second time interval ${\text{Δt}}_{\text{preII}} {\text{= Δt}}_{\text{pre}}$ is started. If using said counter incrementing from time t₁ = 0 to time t₂, said counter starts decrementing at time t₂ down to zero.

During second time interval Δt_{preII}, the cuff is deflated down to pₘᵢₙ at time t_{dz}. The pressure pₘᵢₙ is in the present embodiment 0,66 kPa (5 mmHg). At time t_{dz}, a death-time interval Δt_{dea} is started, said death-time interval Δt_{dea} ends at time tₙₑₓₜ, said time tₙₑₓₜ is identical with the end of second time interval Δt_{preII}. At time tₙₑₓₜ, a next subsequent measuring cycle is initialled by inflating cuff up to pressure pₘₐₓ. Thereafter, a further stepwise deflating of cuff occurs as above described. The cuff works in the stat mode and, therefore, a array of subsequently following cycles are conducted.

The pressure threshold value pₜₕᵣ corresponds to the mean blood pressure pₘₑₐ of the subject to be measured.

Since, during the first measuring cycle, the mean pressure value is not known, an incrementing of counter starts if cuff pressure reaches minimum threshold pressure value pₜₕᵣₘᵢₙ. Therefore, the start point of the incrementing of first counter at time t_{1f} is somewhat earlier than start time t₁. The deviation, however, is very small because of the great gradient of cuff pressure within the inflating step.

The area of possible threshold pressure values pₜₕᵣ being the base for the start of time interval Δtₚᵣₑ are limited by maximum and minimum threshold values pₜₕᵣₘₐₓ and pₜₕᵣₘᵢₙ, resp., with pₘᵢₙ = 6,6 kPa (50 mmHg) and pₘₐₓ = 13,33 kPa (100 mmHg).

As a result, the death-time interval Δt_{dea} is both a function of the mean pressure value, if ${\text{p}}_{\text{thr}} {\text{= p}}_{\text{mea}}$ 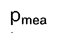 and of the time between two subsequent incremental deflating steps within one individual measuring cycle.

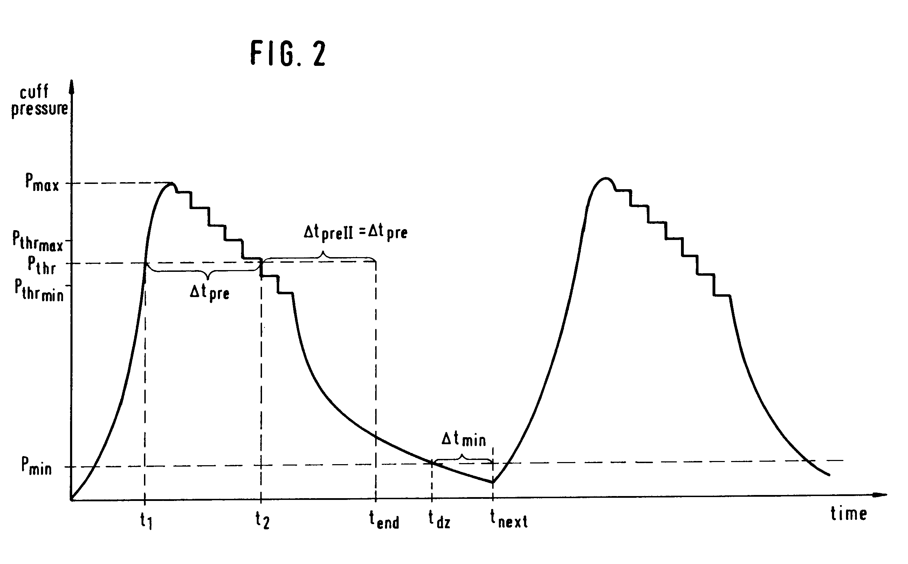

Referring now to Figure 2, showing a graph similar to that of Figure 1 but illustrating a situation wherein in second time interval Δt_{preII} ends before cuff pressure during deflating steps has reached pₘᵢₙ.

This situation may arise if, for example, the mean pressure value is very high and the time interval Δtₚᵣₑ very short.

A start of a new cuff inflating step at time t_{end}, e.g. after interval Δt_{preII} may result in many measurement cycles per time unit. However, based on the fact that the cuff pressure cannot reach pₘᵢₙ, the subject to be measured always has the feeling of an inflated compressed cuff around its limb. This compressed situation or feeling is very disagreeable, in particular within stat mode, and may result in blood accumulation within the at least partial occluded artery.

Therefore, the cuff pressure is allowed to be reduced at least to pₘᵢₙ.

Within the in Figure 2 illustrated embodiment of the invention, a minimum death-time interval Δtₘᵢₙ is provided, for example 2 seconds.

It is possible to reduce the death-time interval Δtₘᵢₙ to zero, in particular if the graph branch between time t_{end} end time t_{dz} is very flat and approximates to pₘᵢₙ. In these cases, a next measuring cycle can be started immediately after cuff pressure reaches pₘᵢₙ.

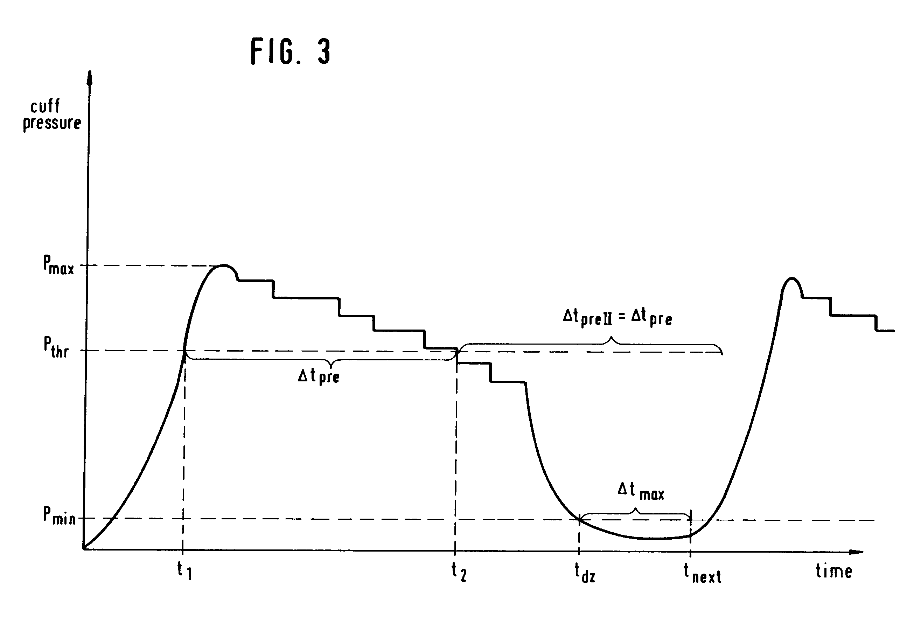

Referring now to Figure 3, a situation is illustrated wherein the time interval Δtₚᵣₑ is so long that said second time interval Δt_{preII} ends after a long interval after cuff pressure has reached pₘᵢₙ. If starting a next subsequent measuring cycle only after end of second time interval Δt_{preII}, an unnecessary long death-time interval will result.

Therefore, a maximum death-time interval Δtₘₐₓ, for example 6 seconds, is provided. A next subsequent measuring cycle is started at time tₙₑₓₜ after maximum death-time interval Δtₘₐₓ has gone, also if second time interval Δt_{preII} has not yet ended.

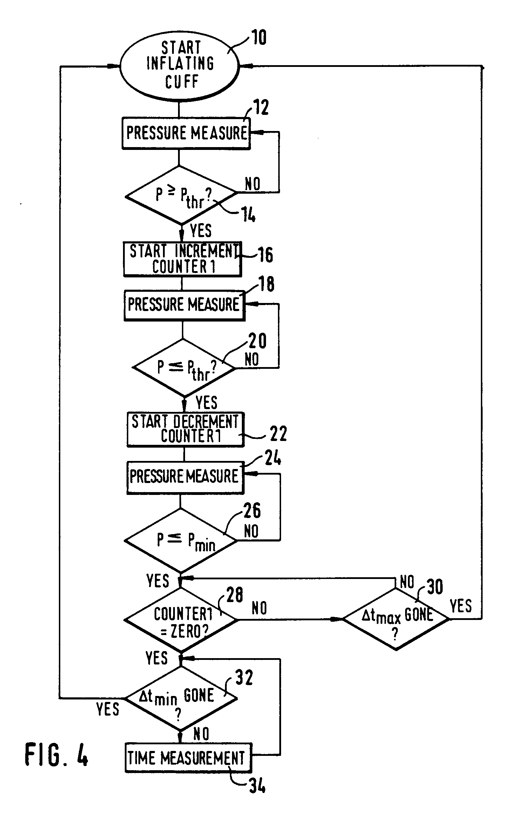

The principles underlying the operation of the apparatus of the present invention are best described with reference to the flow chart in Figure 4 to which attention should now be directed.

To this end, proceeding from a start block 10 a step 12 detects the actual cuff pressure. Test 14 determines whether the pressure value is equal or above to a predetermined threshold pressure pₜₕᵣ. If pₜₕᵣ is not yet reached, test 12 determines actual cuff pressure again ("NO" branch of test 14). However, if actual cuff pressure has reached pₜₕᵣ, block 16 starts incrementing counter 1 ("YES" branch of test 14). Subsequently, step 18 determines the actual cuff pressure. Test 20 determines whether actual cuff pressure has decreased down to pₜₕᵣ again. If threshold pressure pₜₕᵣ is not yet reached, step 18 further conducts pressure measurements ("NO" branch of test 20). However, if actual pressure p has reached threshold pressure pₜₕᵣ, block 22 stops incrementing counter 1 and starts decrementing counter 1 ("YES" branch of test 20). Step 24 determines actual cuff pressure and test 26 determines whether actual cuff pressure has reached pₘᵢₙ. If pₘᵢₙ is not reached, step 24 determines actual pressure again ("NO" branch of test 26). However, if pₘᵢₙ is already reached test 28 determines whether counter 1 = zero ("YES" branch of test 26). If counter 1 is not yet zero, test 30 determines whether maximum death-time interval Δtₘₐₓ has gone ("NO" branch of test 28). If maximum death-time interval has gone, a new inflating of cuff is started by start 10 ("YES" branch of test 30). This situation is illustrated within Figure 3.

However, if test 30 determines Δtₘₐₓ has not gone, test 28 determines whether counter 1 is zero ("NO" branch of test 30).

However, if test 28 determines counter 1 = zero, test 32 determines whether minimum death-time interval Δtₘᵢₙ has gone. If minimum death-time interval has ended, start 10 initiates a next subsequent cuff inflating step ("YES" branch of test 32). This situation is illustrated within Figure 1.

However, if test 32 determines tₘᵢₙ is not gone, a step 34 conducts time measurements until test 32 indicates that Δtₘᵢₙ has gone. This situation is illustrated within Figure 2.

The time measurement of test 32 may occur in connection with a second counter. Said second counter starts incrementing if actual cuff pressure value has reached pₘᵢₙ. Step 34 determines whether second counter has incremented up to Δtₘᵢₙ. Said second counter also can be used for determining whether Δtₘₐₓ has ended.

## Claims

1. A method for measuring blood pressure, comprising the steps of
- applying a blood pressure cuff about a subject's limb containing an artery;
- inflating said cuff to a pressure pₘₐₓ about the systolic pressure p_{sys}, thereby occluding said artery;
- reducing cuff pressure from pₘₐₓ to at least a pressure pₘᵢₙ below the diastolic pressure p_{dia}, thereby permitting an increasing flow through the progressively less occluded artery;
- detecting of effects at the cuff caused by said increasing flow through said progressively less occluded artery;
- processing said detected effects in processing means and displaying said processed effects as subject's actual blood pressure values, and
- conducting a next subsequent blood pressure measuring cycle after the end of a death-time interval Δt_{dea}, said death-time interval starts at a time t_{dz} after cuff pressure reaching pₘᵢₙ,
characterized by
- starting said next subsequent blood pressure measuring cycle at a time tₙₑₓₜ being a function of a cycle specific time interval Δtₚᵣₑ of the preceding measuring cycle.

2. A method according to claim 1, characterized in that the time interval Δtₚᵣₑ starts at a time t₁ after reaching a threshold cuff pressure pₜₕᵣ during inflating said cuff, and ends at a time t₂ after reaching the said threshold pressure pₜₕᵣ again during the cuff pressure reducing the step of said cycle.

3. A method according to claim 2, characterized in that the said threshold pressure pₜₕᵣ corresponds to the mean blood pressure pₘₑₐ.

4. A method according to claim 2 or 3, characterized in that the said threshold cuff pressure pₜₕᵣ is limited as
${\text{p}}_{\text{thrmin}} {\text{≦ p}}_{\text{thr}} {\text{≦ p}}_{\text{thrmax}} \text{.}$

5. A method according to claim 4, characterized in that for measuring the blood pressure of a human, pₜₕᵣ is
${\text{p}}_{\text{thrmin}} {\text{= 6,66 kPa(50 mmHg) ≦ p}}_{\text{thr}} {\text{≦ p}}_{\text{thrmax}} \text{= 13,33 kPa(100 mmHg).}$

6. A method according to any of the preceding claims, characterized in that a next subsequent cycle is started if a second time interval ${\text{Δt}}_{\text{preII}} {\text{= Δt}}_{\text{pre}}$ 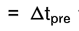 following to the first time interval Δtₚᵣₑ has ended.

7. A method according to claim 6, characterized in that said second cycle is started if both, said second time period Δt_{preII} and a minimum death-time interval Δtₘᵢₙ have ended.

8. A method according to claim 6 or 7, characterized in that said second cycle is started if a maximum death-time interval Δtₘₐₓ has ended, but said second time interval t_{preII} has not yet ended.

9. An apparatus for automated blood pressure measuring, in particular for cyclic measuring in the so-called stat mode, comprising
- an inflatable and deflatable pressure cuff, said cuff being applicable about a subject's limb containing an artery;
- means for inflating said cuff to a pressure pₘₐₓ above the systolic pressure p_{sys}, thereby occluding said artery;
- means for reducing cuff pressure from pₘₐₓ to at least a pressure pₘᵢₙ below the diastolic pressure p_{dia}, thereby permitting an increasing flow through the progressively less occluded artery;
- means for detecting of effects at the cuff caused by said increasing flow through said progressively less occluded artery;
- a processing means for processing said detected effects and for displaying said processed effects as subject's actual blood pressure values, said processing means further comprise means for retarding start of a next measuring cycle until a death-time interval Δt_{dea} has ended,
characterized in that
said means for retarding start of the next cycle comprise time incrementing means for storing a cycle specific time interval Δtₚᵣₑ of a preceding measuring cycle, and that said retarding means allow a start of a next subsequent cycle as a function of said time interval Δtₚᵣₑ.

10. Apparatus according to claim 9, characterized in that said retarding means comprise a first counter, said first counter starts incrementing after reaching at a time t₁ a threshold cuff pressure pₜₕᵣ during inflating cuff, and said first counter starts decrementing after reaching at a time t₂ the said threshold pressure pₜₕᵣ again during the cuff pressure reducing step of said cycle, and said subsequent next measuring cycle is started if first counter is zero.

11. Apparatus according to claim 10, characterized in that said first counter is connected to a second counter, said second counter increments a preselected death-time interval, said second counter starts incrementing after cuff pressure has reached pₘᵢₙ, and a start of said next subsequent cycle occurs if
first counter has reached zero and meanwhile second counter has incremented to a minimum death-time interval Δtₘᵢₙ,
but the latest if second counter has incremented to a preselected maximum death-time interval Δtₘₐₓ.

## Patentansprüche

1. Verfahren zur Blutdruckmessung, mit den Schritten
- Anlegen einer Blutdruckmanschette um ein eine Arterie enthaltendes Gliedmaß eines Lebewesens;
- Aufpumpen der Manschette auf einen Druck pₘₐₓ oberhalb des systolischen Druckes p_{sys}, wodurch die Arterie verschlossen wird;
- Vermindern des Druckes der Manschette von pₘₐₓ auf zumindest einen Druck pₘᵢₙ unterhalb des diastolischen Druckes p_{dia}, wodurch ein zunehmendes Strömen durch die nach und nach weniger verschlossene Arterie möglich ist;
- Erfassen der durch das zunehmende Strömen durch die nach und nach weniger verschlossene Arterie verursachten Effekte an der Manschette;
- Verarbeiten der erfaßten Effekte in Verarbeitungsmitteln und Anzeigen der verarbeiteten Effekte als aktuelle Blutdruckwerte des Lebewesens, und
- Durchführen eines nachfolgenden weiteren Blutdruckmeßzyklus nach Ende eines Totzeitintervalls Δt_{dea}, wobei das Totzeitintervall zu einem Zeitpunkt t_{dz} beginnt, nachdem der Manschettendruck pₘᵢₙ erreicht hat,
dadurch gekennzeichnet,
- daß der nachfolgende weitere Blutdruckmeßzyklus zu einem Zeitpunkt tₙₑₓₜ beginnt, der eine Funktion eines zyklusspezifischen Zeitintervalls Δtₚᵣₑ des vorangegangenen Meßzyklus ist.

2. Verfahren nach Anspruch 1, dadurch gekennzeichnet, daß das Zeitintervall Δ tₚᵣₑ zu einem Zeitpunkt t₁, nach Erreichen eines Schwellwertmanschettendruckes pₜₕᵣ beim Aufpumpen der Manschette, beginnt und das in einem Zeitpunkt t₂ endet, nachdem der Schwellwertdruck pₜₕᵣ beim Vermindern des Manschettendrucks in diesem Zyklus erneut erreicht wird.

3. Verfahren nach Anspruch 2, dadurch gekennzeichnet, daß der Schwellwertdruck pₜₕᵣ dem Mean-Blutdruck pₘₑₐ entspricht.

4. Verfahren nach Anspruch 2 oder 3, dadurch gekennzeichnet, daß der Schwellwertmanschettendruck pₜₕᵣ wie folgt begrenzt ist:
${\text{p}}_{\text{thrmin}} {\text{≦ p}}_{\text{thr}} {\text{≦ p}}_{\text{thrmax}} \text{·}$

5. Verfahren nach Anspruch 4, dadurch gekennzeichnet, daß beim Blutdruckmessen eines Menschen pₜₕᵣ
${\text{}}_{\text{Pthrmin}} {\text{=6,66 kPa(50mmHg) ≦ p}}_{\text{thr}} {\text{≦ p}}_{\text{thrmax}} \text{=13,33 kPa(100mmHg)}$
ist.

6. Verfahren nach einem der vorhergehenden Ansprüche, dadurch gekennzeichnet, daß ein nachfolgender weiterer Zyklus dann gestartet wird, falls ein zweites Zeitintervall ${\text{Δ t}}_{\text{prell}} {\text{= Δ t}}_{\text{pre}} \text{,}$ 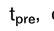 das dem ersten Zeitintervall Δ tₚᵣₑ folgt, geendet hat.

7. Verfahren nach Anspruch 6, dadurch gekennzeichnet, daß der zweite Zyklus gestartet wird, falls sowohl das zweite Zeitintervall Δ tₚᵣₑₗₗ als auch ein Minimum-Totzeitintervall Δ tₘᵢₙ geendet haben.

8. Verfahren nach Anspruch 6 oder 7, dadurch gekennzeichnet, daß der zweite Zyklus beginnt, wenn ein Maximum-Totzeitintervall Δ tₘₐₓ geendet hat, selbst wenn das zweite Zeitintervall Δ tₚᵣₑₗₗ noch nicht geendet hat.

9. Vorrichtung zur automatischen Blutdruckmessung, insbesondere für zyklische Messungen im sogenannten Stat Mode, mit
- einer aufpumpbaren und ablaßbaren Druckmanschette, wobei die Manschette um ein eine Arterie enthaltendes Gliedmaß eines Lebewesens legbar ist;
- Mittel zum Aufpumpen der Manschette auf einen Druck pₘₐₓ oberhalb des systolischen Druckes p_{sys}, wodurch die Arterie verschlossen wird;
- Mittel zum Vermindern des Manschettendruckes von pₘₐₓ auf zumindest einen Druck pₘᵢₙ unterhalb des diastolischen Druckes p_{dia}, wodurch ein zunehmendes Strömen durch die nach und nach weniger verschlossene Arterie möglich ist;
- Mittel zum Erfassen der durch das zunehmende Strömen durch die nach und nach weniger verschlossene Arterie verursachten Effekte an der Manschette;
- Verarbeitungsmittel zum Verarbeiten der erfaßten Effekte und zum Anzeigen der verarbeiteten Effekte als aktuelle Blutdruckwerte des Lebewesens, wobei die Verarbeitungsmittel ferner Mittel zum Verzögern des Beginns eines nächsten Meßzyklus bis ein Totzeitintervall Δ t_{dea} geendet hat, aufweisen,
dadurch gekennzeichnet,
- daß die Mittel zum Verzögern des Beginns eines nächsten Zyklus Zeitinkrementiermittel zum Speichern eines zyklusspezifischen Zeitintervalls Δ tₚᵣₑ eines vorangegangenen Meßzyklus aufweisen, und daß die Verzögerungsmittel den Beginn eines nachfolgenden weiteren Zyklus als Funktion des Zeitintervalls Δ tₚᵣₑ ermöglichen.

10. Vorrichtung nach Anspruch 9, dadurch gekennzeichnet, daß die Verzögerungsmittel einen ersten Zähler aufweisen, daß der erste Zähler zu inkrementieren beginnt, nachdem zu einem Zeitpunkt t₁ ein Schwellwertmanschettendruck pₜₕᵣ beim Aufpumpen der Manschette erreicht wurde, und daß der erste Zähler zu dekrementieren beginnt, nachdem zu einem Zeitpunkt t₂ dieser Schwellwertdruck pₜₕᵣ während des Schrittes der Verminderung des Manschettendrucks dieses Zyklus erneut erreicht wird, und daß der nachfolgende weitere Meßzyklus beginnt, wenn der erste Zähler bei null ist.

11. Vorrichtung nach Anspruch 10, dadurch gekennzeichnet, daß der erste Zähler mit einem zweiten Zähler verbunden ist, daß der zweite Zähler ein vorbestimmtes Totzeitintervall inkrementiert, daß der zweite Zähler zu inkrementieren beginnt, nachdem der Manschettendruck pₘᵢₙ erreicht hat, und daß ein nachfolgender weiterer Zyklus beginnt, wenn
- der erste Zähler null erreicht hat und zwischenzeitlich der zweite Zähler auf ein Minimum-Totzeitintervall Δ tₘᵢₙ inkrementiert hat, jedoch spätestens, wenn der zweite Zähler auf ein vorbestimmtes Maximum-Totzeitintervall Δ tₘₐₓ inkrementiert hat.

## Revendications

1. Procédé pour la mesure de la pression sanguine, comportant les étapes de
- application d'une manchette de pression sanguine autour d'un membre du patient contenant une artère;
- gonflage de ladite manchette jusqu'à une pression pₘₐₓ supérieure à la pression systolique p_{sys}, bouchant ainsi ladite artère;
- réduction de la pression de la manchette depuis pₘₐₓ jusqu'à, au moins, une pression pₘᵢₙ inférieure à la pression diastolique p_{dia}, autorisant ainsi un débit croissant à travers l'artère progressivement de moins en moins bouchée;
- détection des effets sur la manchette dus au débit croissant à travers l'artère progressivement de moins en moins bouchée;
- traitement desdits effets détectées par un moyen de traitement et affichage desdits effets traitées comme valeurs de la pression sanguine réelle du patient, et
- commande du cycle de mesure suivant de la pression sanguine, à l'issue d'un temps mort Δ t_{dea}, ledit temps mort commençant à l'instant t_{d2} après avoir atteint la pression de la manchette pₘᵢₙ.
caractérisé par
- le début dudit cycle de mesures suivant de la pression sanguine à un instant tₙₑₓₜ qui est une fonction de la durée spécifique du cycle Δ tₚᵣₑ du cycle de mesure précédent.

2. Procédé selon la revendication 1, caractérisé en ce que la durée Δ tₚᵣₑ commence à l'instant t₁ après avoir atteint une pression de seuil de la manchette pₜₕᵣ durant le gonflage de ladite manchette, et se termine à un instant t₂ après avoir atteint à nouveau ladite pression de seuil pₜₕᵣ durant l'étape de diminution de la pression de la manchette dudit cycle.

3. Procédé selon la revendication 2, caractérisé en ce que ladite pression de seuil Δ tₚᵣₑ correspond à la pression sanguine moyenne pₘₑₐ.

4. Procédé selon la revendication 2 oui 3, caractérisé en ce que ladite pression de seuil de la manchette Pₜₕᵣ est limitée selon
${\text{p}}_{\text{thrmin}} {\text{≦ p}}_{\text{thr}} {\text{≦ p}}_{\text{thrmax}} \text{.}$

5. Procédé selon la revendication 4, caractérisé en ce que pour mesurer la pression sanguine d'un humain, pₜₕᵣ vaut
${\text{p}}_{\text{thrmin}} {\text{=6,6 kPa(50mmHg)≦p}}_{\text{thr}} {\text{≦p}}_{\text{thrmax}} \text{=13,33 kPa(100mmHg).}$

6. Procédé selon l'une quelconque des revendications précédentes, caractérisé en ce qu'un autre cycle commence si un seconde durée ${\text{Δ t}}_{\text{preII}} {\text{= Δ t}}_{\text{pre}}$ 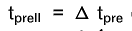 qui suit la première durée Δ tₚᵣₑ, est terminée.

7. Procédé selon la revendication 6, caractérisé en ce que ledit second cycle commence si, à la fois ladite seconde durée Δ t_{preII} et un temps mort minimum Δ tₘᵢₙ sont achevés.

8. Procédé selon la revendication 6 ou 7, caractérisé en ce que ledit second cycle commence lorsqu'un temps morte maximum Δ tₘₐₓ s'est achevé, mais que ladite seconde durée Δ t_{preII} n'est pas encore terminée.

9. Appareil pour la mesure automatique de la pression sanguine, en particulier pour la mesure cyclique dans le mode dit stat, comportant
- une manchette à pression gonflable et dégonflable, ladite manchette pouvant s'appliquer autour d'un membre du patient, contenant une artère;
- un moyen de gonflage de ladite manchette jusqu'à une pression pₘₐₓ supérieure à la pression systolique p_{sys}, bouchant ainsi ladite artère;
- un moyen de réduction de la pression de la manchette depuis pₘₐₓ jusqu'à, au moins, une pression pₘᵢₙ inférieure à la pression diastolique p_{dia}, autorisant ainsi un débit croissant à travers l'artère progressivement de moins en moins bouchée;
- un moyen de détection des effets sur la manchette, provoqués par ledit débit croissant à travers l'artère progressivement de moins en moins bouchée;
- un moyen de traitement destiné à traiter lesdits effets détectés et à afficher lesdits effets traités comme valeurs de la pression sanguine réelle du patient, ledit moyen de traitement comportant également un moyen destiné à retarder le début du cycle de mesure suivant jusqu'à ce que le temps mort Δ t_{dea} soit terminé.
caractérisé en ce que
ledit moyen destiné à retarder le début du cycle suivant comporte un moyen d'incrémenter le temps afin de stocker la durée spécifique du cycle Δ tₚᵣₑ d'un cycle de mesure précédent, et ledit moyen de retard autorise le début du cycle suivant en fonction de ladite durée Δ tₚᵣₑ.

10. Appareil selon la revendication 9, caractérisé en ce que ledit moyen de retard comporte un premier compteur, ledit premier compteur commençant l'incrémentation après avoir atteint, à un instant t₁, la pression de seuil de la manchette pₜₕᵣ, au cours du gonflage de la manchette et ledit premier compteur commence la décrémentation après avoir atteint à un instant t₂ ladite pression de seuil de la manchette pₜₕᵣ, durant à nouveau l'étape de réduction de la pression de la manchette dudit cycle et ledit cycle de mesures suivant commence si le premier compteur est à zéro.

11. Appareil selon la revendication 10, caractérisé en ce que ledit premier compteur est relié à un second compteur, ledit second compteur incrémente un temps mort maximum présélectionné, ledit second compteur commence l'incrémentation après que la pression de la manchette ait atteint pₘᵢₙ, et le début du cycle suivant a lieu si
le premier compteur a atteint zéro et, dans l'intervalle, le second compteur s'est incrémenté jusqu'à un temps mort minimum Δ tₘᵢₙ,
mais il s'agit du dernier si le second compteur s'est incrémenté jusqu'à un temps mort maximum présélectionné Δ tₘₐₓ.
